(19) Europäisches Patentamt
European Patent Office
Office européen des brevets

(11) **EP 1 679 378 B1**

(12) **EUROPEAN PATENT SPECIFICATION**

(45) Date of publication and mention
of the grant of the patent:
**22.03.2017 Bulletin 2017/12**

(51) Int Cl.:
***C12P 21/02*** *(2006.01)*      ***C12P 21/06*** *(2006.01)*

(21) Application number: **04772984.3**

(86) International application number:
**PCT/JP2004/013302**

(22) Date of filing: **13.09.2004**

(87) International publication number:
**WO 2005/028660 (31.03.2005 Gazette 2005/13)**

(54) **PROCESS FOR PRODUCING ALPHA-GLYCATED DIPEPTIDES BY PROTEOLYSIS**

VERFAHREN ZUR HERSTELLUNG VON ALPHA-GLYKIERTEN DIPEPTIDEN DURCH PROTEOLYSE

PROCÉDÉ DE PRODUCTION DE DIPEPTIDES ALPHA-GLYQUÉS PAR PROTÉOLYSE

(84) Designated Contracting States:
**DE FR GB IT**

(30) Priority: **18.09.2003 JP 2003326224**
**19.12.2003 JP 2003421755**

(43) Date of publication of application:
**12.07.2006 Bulletin 2006/28**

(73) Proprietor: **KIKKOMAN CORPORATION**
**Noda-shi,**
**Chiba 278-8601 (JP)**

(72) Inventors:
• **HIROKAWA, Kozo,**
**c/o Kikkoman Corporation**
**Noda-shi,**
**Chiba 2788601 (JP)**
• **KUROSAWA, Keiko,**
**c/o Kikkoman Corporation**
**Noda-shi,**
**Chiba 2788601 (JP)**
• **KAJIYAMA, Naoki,**
**c/o Kikkoman Corporation**
**Noda-shi,**
**Chiba 2788601 (JP)**

(74) Representative: **Denison, Christopher Marcus et al**
**Mewburn Ellis LLP**
**City Tower**
**40 Basinghall Street**
**London EC2V 5DE (GB)**

(56) References cited:
**EP-A1- 1 223 224      EP-A1- 1 291 416**
**JP-A- 2001 095 598**

• **HIROKAWA K.ET AL: 'ENZYMES USED FOR THE DETERMINATION OF HBA1C' FEMS MICROBIOL.LETT. vol. 235, June 2004, pages 157 - 162, XP002903440**
• **HIROKAWA K ET AL: 'MOLECULAR CLONING AND EXPRESSION OF NOVEL FRUCTOSYL PEPTIDE OXIDASES AND THEIR APPLICATION FOR THE MEASUREMENT OF GLYCATED PROTEIN' BIOCHEM. BIOPHYS.RES.COMMUN. vol. 311, November 2003, pages 104 - 111, XP004465110**
• **DATABASE BRENDA 'Information on EC 3.4.24.27 - thermolysin'**
• **DATABASE BRENDA 'Information on EC 3.4.24.28 - bacillolysin'**
• **'Information on EC 3.4.21.63 - Oryzin' INTERNET ARTICLE, [Online] Retrieved from the Internet: <URL:http://www.brenda-enzymes.info/enzyme.php?ecno=3.4.21.63> [retrieved on 2015-11-13]**

**Description**

Technical Field

The present invention relates to a method for producing α-glycated dipeptide.

Background Art

Glycated protein is nonenzymatically-glycated protein. Specifically, the glycated protein is generated as a result of nonenzymatical covalent bonding of aldehyde group on the sugar side (that is, on the aldose (a monosaccharide potentially having an aldehyde group and its derivative) side) to amino group on the protein side. Furthermore, such glycated protein is formed when a Schiff base generated as a reaction intermediate is subjected to Amadori rearrangement. Thus, the glycated protein is also referred to as so-called Amadori compound.

The glycated protein is contained in body fluids such as *in vivo* blood or biological samples such as hair. The concentration of the glycated protein existing in blood strongly depends on the concentration of saccharides such as glucose, which are dissolved in sera. Under diabetic conditions, glycated protein generation is enhanced. Furthermore, the concentration of glycated hemoglobin contained in erythrocytes or the concentration of glycated albumin in sera reflects a past average blood glucose level for a certain time period. Hence, determination of the amount of such glycated protein is important for diagnosing or controlling the symptoms of diabetes.

Glycated hemoglobin (hereinafter, abbreviated as HbAlc.) is fructosyl protein having a structure generated through nonenzymatic binding of glucose to the N-terminal amino acid of a hemoglobin β-subunit so as to form a Schiff base, resulting in the binding of fructose through Amadori rearrangement. Such HbAlc clinically reflects an average blood glucose level for the past 1 to 2 months. Thus, HbAlc is important as an index for controlling diabetes and rapid and precise determination methods therefor are required.

Currently, as a method for determining the amount of HbAlc, IFCC Practical Standard Methods (see Kobold U., et al, Clin. Chem. 43, 1944-1951 (1997)) disclose a determination method that involves hydrolysing (treatment at 37°C for 18 hours) HbAlc with endoprotease Glu-C, separating the hexapeptide fragment obtained from N-terminus of its β chain by HPLC, and determining the amount of the resultant using a capillary electrophoresis method or a mass spectrometry method, for example. However, the method is problematic in that it requires a special apparatus and complicated procedures and is economically inefficient.

Hence, an enzymatic method has been proposed as a method for determining the amount of HbAlc in a highly precise manner via simple procedures at low cost. Such an enzymatic method involves denaturing glycated protein with protease, causing fructosyl amino acid oxidase to act on liberated glycated amino acid, and then determining the amount of the thus generated hydrogen peroxide Examples of oxidase, which have been disclosed for use in such an enzymatic determination method, include oxidase produced by bacteria of the genus *Corynebacterium* (see JP Patent Publication (Kokoku) No. 5-33997 B (1993) and JP Patent Publication (Kokoku) No. 6-65300 B (1994)), oxidase produced by strains of the genus *Aspergillus* (see JP Patent Publication (Kokai) No. 3-155780 A (1991)), oxidase produced by strains of the genus *Gibberella* (see JP Patent Publication (Kokai) No. 7-289253 A (1995)), oxidase produced by strain of the genus *Fusarium* (see JP Patent Publication (Kokai) No. 7-289253 A (1995) and JP Patent Publication (Kokai) No. 8-154672 A (1996)), oxidase produced by strains of the genus *Penicillium* (see JP Patent Publication (Kokai) No. 8-336386 A (1996)), and ketoamine oxidase (see JP Patent Publication (Kokai) No. 5-192193 A (1993)). Furthermore, the following methods (a) to (i) have been thus far known as examples, wherein α-glycated amino acid (the α-amino group of amino acid has been glycated) is liberated from hemoglobin having glycated N-terminal amino acid:

(a) a method that involves adding 8M urea to glycohemoglobin, boiling the mixture for 20 minutes for denaturation, carrying out trypsin treatment, and then determining the amount of the resultant with fructosyl amino acid oxidase (FAOD) derived from the genus *Penicillium* (see JP Patent Publication (Kokai) No. 8-336386 A (1996));
(b) a method that involves treating glycohemoglobin with protease and then determining the amount of the resultant with FAOD derived from the genus *Aspergillus* (see JP Patent Publication (Kokai) No. 10-33177 A (1998) and JP Patent Publication (Kokai) No. 10-33180 A (1998));
(c) a method that involves determining the amount of glycated hemoglobin using endoprotease and exoprotease (see International Patent Publication No. 97/13872 pamphlet);
(d) a method that involves enzymatically treating peptide or protein having fructosyl N-terminal valine using serine carboxypeptidase (see JP Patent Publication (Kokai) No. 2001-57897 A);
(e) a method that involves carrying out treatment using protease capable of cleaving the carboxyl group side of the third leucine from the β chain N-terminus of HbAlc, treating the resultant with protease capable of excising histidyl leucine from the generated fructosyl valyl-histidyl-leucine, and then determining the amount of hemoglobin Alc (see JP Patent Publication (Kokai) No. 2000-300294 A);

(f) a method that involves liberating glycated amino acid using novel enzymes derived from the genus *Corynebacterium* and the genus *Pseudomonas* (such enzymes being capable of liberating amino acid with glycated $\alpha$-amino group from glycated protein) and then determining the amount of the resultant (see International Patent Publication No. 00/50579 pamphlet);

(g) a method that involves liberating glycated amino acid using novel enzymes derived from the genera *Sphingobacterium, Sphingomonas, Comamonas, Mucor,* and *Penicillium* (such enzymes being capable of liberating amino acid with glycated $\alpha$-amino group from glycated protein) and then determining the amount of the resultant (see International Patent Publication No. 00/61732 pamphlet);

(h) a method that involves treating a sample containing protein with protease in the presence of a tetrazolium compound, causing the thus obtained proteolysed product to react with FAOX, and then rapidly determining the amount of glycated protein (see International Patent Publication No. 02/27012 pamphlet); and

(i) a method that involves causing deblocking aminopeptidase, dipeptidyl aminopeptidase, leucine aminopeptidase, N-acylaminoacyl-peptide hydrolase, or hemicellulase to act on a test solution containing N-terminal-glycated peptide or protein, liberating the N-terminal-glycated amino acid, and then determining the amount of the thus generated glycated amino acid (see JP Patent Publication (Kokai) No. 2002-315600 A).

However, according to experiments carried out by the present inventors, there are no examples wherein $\alpha$-glycated amino acid could have been liberated even by causing various proteases to act on HbAlc. Specifically, various proteases cannot cleave HbAlc into sizes smaller than that of $\alpha$-glycated peptide. Almost no $\alpha$-glycated amino acid can be cleaved with such proteases. It has been concluded that the amount of HbAlc cannot be determined with good sensitivity as long as the above-mentioned fructosyl amino acid oxidases are used. As described above, for HbAlc determination, a good method for determining the amount of HbAlc with the highest sensitivity is a determination method that involves detecting $\alpha$-glycated peptide or preferably $\alpha$-glycated dipeptide that is liberated through protease treatment using oxidase that acts on such peptide or dipeptide as a substrate. Such oxidase acting on $\alpha$-glycated dipeptide and protease capable of excising glycated peptide has already been disclosed in JP Patent Publication (Kokai) No. 2001-95598 A and JP Patent Publication (Kokai) No. 2003-235585 A. However, to realize more rapid HbAlc determination with higher sensitivity, protease having higher activity of excising $\alpha$-glycated dipeptide has been required.

Hence, an object to be achieved by the present invention is to provide a method for producing $\alpha$-glycated dipeptide, by which $\alpha$-glycated dipeptide (glycated dipeptide wherein the $\alpha$-amino group of the N-terminal amino acid of the dipeptide have been glycated) are efficiently liberated from glycated protein or glycated peptide through a kind of protease treatment.

Disclosure of the Invention

As a result of intensive studies to achieve the above objects, the present inventors have discovered that $\alpha$-glycated dipeptide (glycated dipeptide wherein the $\alpha$-amino group of N-terminal amino acid of dipeptide have been glycated) can be efficiently liberated from glycated protein or glycated peptide through a kind of protease treatment. Thus, the present inventors have completed the present invention. The present inventors have also discovered that glycated protein or glycated peptide can be determined in a highly precise manner with simple procedures within a short time period through determination of the amount of liberated $\alpha$-glycated peptide using the above oxidase.

The present invention is to provide the following inventions:

(1) a method for producing $\alpha$-glycated dipeptide, which comprises causing a protease to act on an N-terminal-glycated peptide or an N-terminal-glycated protein,
wherein said protease is at least one selected from the group consisting of *Aspergillus* protease P, *Aspergillus* protease M, *Aspergillus* protease A, *Bacillus* dispase, *Bacillus* proteinase N, and *Bacillus* protease S;

(2) the method for producing $\alpha$-glycated dipeptide according to (1), wherein the N-terminal - glycated peptide is fructosyl Val-His-Leu-Thr-Pro-Glu;

(3) the method for producing $\alpha$-glycated dipeptide according to (1), wherein the N-terminal - glycated protein is glycated hemoglobin;

(4) the method for producing $\alpha$-glycated dipeptides according to (1) to (3), wherein the $\alpha$-glycated dipeptide is fructosyl valyl histidine.

Also disclosed herein (but not claimed) are methods for determining the amount of $\alpha$-glycated dipeptide, which comprises causing fructosyl peptide oxidase to act on the $\alpha$-glycated dipeptide obtained by the production method according to (1) to (4) and then determining the amount of the generated hydrogen peroxide.

The present invention will be explained in detail as follows. This application claims priority of Japanese patent application No. 2003-326224 filed on September 18, 2003, and of Japanese patent application No. 2003-421755 filed on December 19, 2003,

N-terminal-glycated protein in the present invention may be any protein, as long as it is generated by nonenzymatic binding of protein to aldose such as glucose.

Examples of glycated protein derived from living bodies include glycoalbumin and HbAlc. For example, the methods disclosed herein may be appropriately used for determining the amounts of HbAlc and the like. Furthermore, examples of N-terminal-glycated peptide in the present invention include not only peptide that is generated by nonenzymatic binding of peptide contained in a sample to aldose such as glucose, but also include peptide generated by enzymatic (e.g., protease and peptidase) or nonenzymatic (e.g., physical shock and heat) cleavage of the above N-terminal-glycated protein. Such glycated protein or glycated peptide is also contained, in general foods such as juices, candies, seasonings, and powdered .foods. Samples containing glycated protein or glycated peptide may be any samples, as long as they contain the above glycated protein or glycated peptide. Examples of such samples include *in vivo* samples such as body fluids (e.g., blood and saliva) and hair. Further examples of such samples include the above foods and the like. These samples may be directly subjected to determination or indirectly subjected to the same after filtration, dialysis treatment, or the like. Furthermore, for example, glycated protein or glycated peptide, the amount of which should be determined, may be appropriately condensed, extracted, and then diluted with water, buffer, or the like.

Proteases that can be used in the present invention are as defined in claims. Enzymes for use in the invention are capable of acting on the above glycated protein or glycated peptide and then liberating $\alpha$-glycated dipeptide. Examples of protease that is capable of efficiently liberating $\alpha$-glycated dipeptide include: proteases derived from *Aspergillus,* such as "IP enzyme, AO protease, peptidase, and molsin (all produced by KIKKOMAN CORPORATION)," "protease A5 (produced by KYOWAKASEI CO.,LTD.)," "umamizyme, protease A, protease M, and protease P (all produced by Amano Enzyme Inc.)," "sumizyme MP, sumizyme LP-20, sumizyme LPL, and sumizyme AP (all produced by Shin Nihon Chemical Co. Ltd.)," and "proteinase 6 (produced by Fluka)"; enzymes derived from *Rhizopus,* such as "peptidase R (produced by Amano Enzyme Inc.); proteases derived from *Bacillus*, such as "dispase (produced by Roche)," "subtilisin (produced by Boehringer Mannheim Corporation)," "proteinase N (produced by Fluka)," "proteinase Type VII (produced by Sigma-Aldrich Corporation)," "proteinase (Bacterial) (produced by Fluka)," "protease N, proleather FG-F, and protease S (all produced by Amano Enzyme Inc.)," "proteinase Type X (produced by Sigma-Aldrich Corporation)," "thermolysin (produced by DAIWA KASEI K.K.)," "pronase E (produced by Kaken Pharmaceutical Co., Ltd.)," and "neutral protease (produced by TOYOBO., LTD.)"; proteases derived from *Streptomyces,* such as "pronase (produced by Boehringer Mannheim Corporation)," "proteinase Type XIV (produced by Sigma-Aldrich Corporation)," and "alkaline protease (produced by TOYOBO., LTD.)"; protease derived from *Tritirachium,* such as "proteinase K (produced by Roche and Wako Pure Chemical Industries, Ltd.)"; protease derived from *Staphylococcus,* such as "Glu-C (produced by Boehringer Mannheim Corporation)"; proteases derived from plants, such as papain (produced by Roche, Wako Pure Chemical Industries, Ltd., Sigma-Aldrich Corporation, Amano Enzyme Inc., and ASAHI FOOD & HEALTHCARE, LTD.)," "ficin (produced by Sigma-Aldrich Corporation)," "bromelain (produced by Amano Enzyme Inc. and Sigma-Aldrich Corporation)"; and proteases derived from animals, such as "pancreatin (produced by Wako Pure Chemical Industries, Ltd.)" and "cathepsin B (produced by Sigma-Aldrich Corporation). Samples containing these proteases can be used. The above proteases may be used independently or 2 or more types thereof may be used in combination. For example, regarding HbAlc, it has been shown that $\alpha$-glycated hexapeptide (fructosyl Val-His-Leu-Thr-Pro-Glu) is generated using endoproteinase Glu-C (Kobold U., et al, Clin. Chem. 1997, 43: 1944-1951). Accordingly, combining Glu-C with the above protease is an extremely effective method for producing glycated dipeptide from HbAlc.

Treatment conditions for a sample may be any conditions, as long as they are conditions under which protease to be used herein can act on glycated protein, the amount of which is determined, following which $\alpha$-glycated dipeptide can be efficiently liberated within a short time period. The amount of a protease to be used herein is appropriately selected depending on the content of glycated protein in a sample, treatment conditions, or the like. In an example, protease derived from strains of the genus *Aspergillus* (e.g., protease P marketed by Amano Enzyme Inc.) is added at a concentration of 0.5 mg/mL to 50 mg/mL and preferably 1 mg/mL to 20 mg/mL. Furthermore, other proteases may also be appropriately added, if necessary. pH employed upon protease treatment may be non-adjusted pH. Alternatively, to achieve appropriate pH for the action of protease to be used, pH may be adjusted using an appropriate pH adjuster such as hydrochloric acid, acetic acid, sulfuric acid, sodium hydroxide, or potassium hydroxide to pH 2 to pH 9 and preferably pH 3 to pH 8, for example. Treatment may also be carried out within a temperature range between 20°C and 50°C, for example. Depending on an enzyme to be used, treatment may be carried out within a higher temperature range between 45°C and 70°C. Treatment time may be any treatment time sufficient for denaturation of glycated protein. Specifically, treatment may be carried out for 1 to 180 minutes and preferably 2 to 60 minutes. The thus obtained treatment solution may be directly used or indirectly used after appropriate heating, centrifugation, condensation, dilution, or the like, if necessary.

Subsequently, the amount of $\alpha$-glycated dipeptide excised by the above methods is determined.

Any methods may be employed, as long as they enable determination of the amount of $\alpha$-glycated dipeptide. Examples of preferable methods for determining the amount of $\alpha$-glycated dipeptide in a highly precise manner with simple procedures at low cost within a short time period include a method that involves causing oxidase to act on α-glycated dipeptide and a method that uses HPLC.

First, the method that involves causing oxidase to act on α-glycated dipeptide will be explained.

Oxidase is caused to act on the above α-glycated dipeptide and then the amount of a product or a consumed product resulting from such action is determined, thereby allowing determination of the amount of glycated dipeptide by an enzymatic method. As such oxidase, any enzyme can be used, as long as it specifically acts on α-glycated dipeptide so as to catalyze a reaction for generating hydrogen peroxide.

Examples of such enzyme include a fructosyl peptide oxidase produced by *Escherichia coli* DH5α (pFP1) (FERM P-17576) disclosed in JP Patent Publication (Kokai) No. 2001-95598 A and a fructosyl peptide oxidase disclosed in JP Patent Publication (Kokai) No. 2003-235585 A.

In addition to the above examples, an enzyme that specifically acts on α-glycated dipeptide so as to catalyze a reaction for generating hydrogen peroxide can be obtained through searches of microorganisms in the natural world or through searches of enzymes derived from animals or plants. Furthermore, such enzyme obtained through searches is prepared by gene recombinant techniques and the thus obtained recombinant enzyme can also be appropriately used Furthermore, such enzyme can also be obtained by modifying known fructosyl amino acid oxidase and the like. Examples of such known fructosyl amino acid oxidase and the like include oxidases produced by bacteria of the genus *Corynebacterium* (JP Patent Publication (Kohyo) No. 5-33997 B (1993) and JP Patent Publication (Kohyo) No. 6-65300 B (1994)), oxidase produced by strains of the genus *Aspergillus* (JP Patent Publication (Kokai) No. 3-155780 A (1991)), oxidase produced by strains of the genus *Gibberella* (JP Patent Publication (Kokai) No. 7-289253 A (1995)), oxidases produced by strains of the genus *Fusarium* (JP Patent Publication (Kokai) No. 7-289253 A (1995) and JP Patent Publication (Kokai) No. 8-154672 A (1996)), oxidase produced by strains of the genus *Penicillium* (JP Patent Publication (Kokai) No. 8-336386 A (1996)), and ketoamine oxidase (JP Patent Publication (Kokai) No. 5-192193 A (1993)).

To obtain oxidase that acts on α-glycated dipeptide through modification of known fructosyl amino acid oxidase and the like, microorganisms capable of producing the above known fructosyl amino acid oxidase and the like are exposed to ultraviolet rays, X ray, radiation, or the like. Alternatively, such oxidase is caused to come into contact with a mutagenic agent such as ethyl methanesulfonate, N-methyl-N'-nitro-N-nitrosoguanidine, or nitrous acid, so as to carry out mutation treatment. A microorganism that produces oxidase that acts on α-glycated dipeptide is selected from the thus obtained mutated microorganisms. However, in general, oxidase that acts on α-glycated dipeptide can be obtained by introducing mutation into genes (hereinafter, referred to as wild type genes) such as genes of the above known fructosyl amino acid oxidase and the like. Any a wild type gene can also be used for introducing mutation, as long as it is a wild type gene of the above fructosyl amino acid oxidase or oxidase analogous thereto, for example, and it enables obtainment of oxidase that acts on α-glycated dipeptide through introduction of mutation.

The titer of fructosyl peptide oxidase that acts on α-glycated dipeptide can be determined by the following method, for example. Such titer can also be determined by other methods.

(1) Preparation of reagent

Reagent 1 (R1): 1.0 kU of peroxidase (hereinafter abbreviated as POD, produced by KIKKOMAN CORPORATION) and 100 mg of 4-aminoantipyrine (hereinafter abbreviated as 4AA, produced by Tokyo Kasei Kogyo Co., Ltd.) are dissolved in a 0.1 M potassium phosphate buffer (pH 8.0). The resulting solution is prepared to a constant volume of 1 L.

Reagent 2 (R2): 500 mg of TOOS (N-ethyl-N-(2-hydroxy-3-sulfopropyl)-m-toluidine, produced by DOJINDO LABORATORIES) is dissolved in ion exchange water. The resulting solution is prepared to a constant volume of 100 mL.

Reagent 3 (R3): 1.25 g of fructosyl Val-His (MW416, its production method will be described below) is dissolved in ion exchange water. The resulting solution is prepared to a constant volume of 10 mL.

(2) Determination

100 μL of R2 is added to 2.7 mL of R1. 100 μL of an enzyme solution containing a fructosyl peptide oxidase is further added to and mixed well with the solution, followed by 5 minutes of pre-heating at 37°C.

Subsequently, 100 μL of R3 is added to and mixed well with the solution. A change in absorbance at 555nm (difference between absorbance determined before and the same determined after 5 minutes of reaction at 37°C with R3) is determined using a spectrophotometer (U-2000A, produced by Hitachi, Ltd.). In addition, the similar procedures are carried out for a control solution, except that 100 μL of ion exchange water is added instead of 100 μL of R3. A graph is obtained by plotting absorbances reflecting the amounts of pigment generated at various concentrations of the previously prepared standard solutions of hydrogen peroxide. Based on such graph, the amounts of hydrogen peroxide corresponding to changes in absorbance are found. These numerical values are used as activity units in enzyme solutions The amount of enzyme that generates 1 μmol of hydrogen peroxide for 1 minute is determined to be 1 U.

By causing the above fructosyl peptide oxidase to act on α-glycated peptide liberated by the protease treatment of the present invention, the amount of α-glycated peptide in a sample can be determined. Furthermore, by determining the amount of α-glycated peptide in a sample, proteases' efficiencies of excising α-glycated peptide can be compared. The amount of fructosyl peptide oxidase to be used herein depends on the amount of α-glycated peptide contained in a treatment solution. For example, fructosyl peptide oxidase may be added at a final concentration between 0.1 U/mL and 50 U/mL and preferably 1 U/mL to 10 U/mL. The pH used when the oxidase is caused to act may be pH 3 to pH 11 and particularly preferably pH 5 to pH 9, for example. It is preferable to adjust pH using a buffer agent so as to achieve a pH appropriate for determination in view of the optimum pH for fructosyl peptide oxidase. However, the pH is not limited to such pH, as long as the pH enables such oxidase to act. The method for adjusting pH is not particularly limited. Examples of such buffer agent include N-[tris (hydroxymethyl)methyl]glycine; phosphate, acetate, carbonate, tris (hydroxymethyl)-aminomethane, borate, citrate, dimethyl glutamate, tricine, and HEPES. Furthermore, if necessary, the pH of a treatment solution after protease treatment may also be appropriately adjusted at the above pH using a buffer agent.

Action time ranges from 1 to 120 minutes and preferably 1 to 30 minutes, for example, and depends on the amount of glycated peptide to be used as a substrate. Any action time may be employed, as long as it is sufficient for fructosyl peptide oxidase to act on such peptide. Action temperature ranges from 20°C to 45°C, for example. Temperature employed for a general enzyme reaction can be appropriately selected.

The amount of hydrogen peroxide generated by the action of fructosyl peptide oxidase may also be determined by any method. Examples of such methods include an electric method using oxygen electrodes, and preferably, an enzymatic method using peroxidase and a proper chromogenic substrate. For example, it is preferable to carry out determination using an enzymatic method with simple procedures within a short time period. An example of a reagent for determining the amount of hydrogen peroxide by an enzymatic method is composted of a 5 mM to 500 mM and preferably 50 mM to 100 mM buffer agent (preferably pH 4 to pH 10), 0.01 mM to 50 mM and preferably 0.1 mM to 20 mM 4-aminoantipyrine as a chromogenic substrate, 0.1 U/mL to 50 U/mL and preferably 1 U/inL to 20 U/mL peroxidase, and the like.

Examples of a buffer agent to be used include N-[tris(hydroxymethyl)methyl]glycine, phosphate, acetate, carbonate, tris(hydroxymethyl)-aminomethane, borate, citrate, dimethyl glutamate, tricine, and HEPES. Examples of a chromogenic substrate include, in addition to 4-aminoantipyrine, ADOS(N-ethyl-N-(2-hydroxy-3-sulfopropyl)-m-anisidine), ALOS(N-ethyl-N-(2-hydroxy-3-sulfopropyl)aniline), 10-(carboxymethyl-aminocarbonyl)-3,7-bis(dimethylamino)phenothiazine (DA-67), N-(carboxymethyl-aminocarbonyl)-4,4'-bis(dimethylamino)diphenylamine (DA-64). Furthermore, if necessary, various additives including a solubilizing agent, a stabilizing agent, a surfactant (e.g., triton X-100, bridge 35, Tween 80, or cholate), a reducing agent (e.g., dithiothreitol, mercaptoethanol, or L-cysteine), bovine serum albumin, saccharides (e.g., glycerine, lactose, or sucrose), and the like may be appropriately added.

When such determination of the amount of hydrogen peroxide is carried out, in general, it is preferable to simultaneously carry out a step of generating hydrogen peroxide through the action of oxidase. A fructosyl peptide oxidase is preferably added at 0.1 U/mL to 50 U/mL and preferably 1 U/mL to 10 U/mL, for example, to the above reagent for determining the amount of hydrogen peroxide.

These reagents for determination may be used in a dry form or in a state of being dissolved or may also be used in a form of a carrier on a thin film such as paper (e.g., an impregnatable sheet of paper) impregnated with such reagent. Enzymes used in the reagents for determination can also be immobilized by a standard method and then repeatedly used. The temperature for determination ranges from 20°C to 45°C, for example. Such temperature can be appropriately selected from temperatures that are used for general enzyme reactions. The time required for determination can be appropriately selected depending on various determination conditions. For example, such time for determination may range from 0.1 to 60 minutes and particularly preferably 1 to 10 minutes. The degree of color development (the amount of change in absorbance) of the above reagent for determination is determined using a spectrophotometer. The result is compared with a standard absorbance. Thus; the amount of glycated peptide or glycated protein contained in a sample can be determined. A general autoanalyser can also be used for determination.

Subsequently, a method for determining the amount of liberated glycated peptide by HPLC will be described.

A protease treatment solution containing liberated glycated peptide is directly or indirectly used for HPLC determination after centrifugal filtration or membrane filtration of the treatment solution and then appropriate condensation and/or dilution of the resultant, if necessary. HPLC may be any HPLC, as long as it enables determination of the amount of the above glycated peptide.

Examples of reverse phase HPLC columns to be used herein include CAPCEL-PAK C-18 (produced by Shiseido Co., Ltd.), TSKgel ODS80Ts (produced by TOSOH CORPORATION), and Shodex RSpak RP18-415 (produced by SHOWA DENKO K.K.). Examples of ion exchange HPLC columns, to be used herein include TSKgel SP-2SW and TSKgel CM-2SW (produced by TOSOH CORPORATION). After a protease treatment solution is adsorbed to such column, target glycated peptide is eluted using an eluant. An eluant may be any eluant, as long as it is appropriate for determination. Examples of such eluant that is used for a reverse phase column include a mixed solution of acetonitrile containing trifluoroacetic acid and water, a mixed solution of a phosphate buffer and acetonitrile, and a mixed solution of an ammonia aqueous solution and acetonitrile. Examples of such eluant that is used for an ion exchange column include a mixed solution of a phosphate buffer and a NaCl solution and a mixed solution of an acetate buffer and acetonitrile. By the use of such eluant, elution may be carried out stepwise or with gradient. Examples of a preferable eluant include a gradient eluant of 0.1% TFA (trifluoroacetic acid)/water-0.1% TFA/30% acetonitrile, and the like. A column, an eluant, elution conditions (e.g., an elution method, the flow rate of an eluant, and temperature), and the like are appropriately combined. Accordingly, it is preferable to set conditions where the elution peak of target $\alpha$-glycated peptide can be separated so as to be as far as possible from the peaks of other components.

Any method may be employed for detecting glycated peptide eluted using an eluant, as long as it enables detection of glycated peptide. Examples of such method that is employed herein include a method that involves detecting absorbances at wavelengths of 210 nm, 215 nm, and the like a method that involves sampling each detection peak and then subjecting the resultant to mass spectrometry analysis so as to determine the peak of a target molecular amount, a method that involves subjecting an eluted product to thin-layer chromatography, and a method that involves sampling elution fractions with time and then subjecting the fractions to colorimetry using a ninhydrin method or a sugar coloring method. For example, when a method that involves detecting absorbance is employed the elution peak area of glycated peptide detected by a monitor is calculated. The result is compared with the elution peak area of a standard substance, and then the amounts of the glycated peptide and the glycated protein can be determined.

Best Mode of Currying Out the Invention

The present invention will be further described specifically by referring to a production example and examples.

(Production example) Production of glycated dipeptide (outside the scope of the invention)

$\alpha$-glycated dipeptide was produced by the following method.

7.0 g (27.6 mmol) of commercial dipeptide (valyl histidine (Val-His), produced by BACHEM, Switzerland) was dissolved in 14 mL of water. 5.8 mL of acetic acid was added to the solution, and then dissolved at approximately 50°C, followed by clarification. Subsequently, 120 mL of ethanol was added to and mixed with the solution and then 14 g (77.8 mmol) of glucose was added to and sufficiently mixed with the solution.

Subsequently, the solution was subjected to heat treatment at 80°C within a closed vessel for 6 hours during which the solution was occasionally stirred. The reaction solution was browned with time. The reaction solution was sampled with time. After appropriate dilution, the solutions were subjected to reverse phase high performance liquid chromatography analysis, thin-layer chromatography analysis, or mass spectrometry analysis. Thus, the generation of target glycated dipeptide was tested. In general, glycated dipeptide can be obtained at good yields through 6 to 10 hours of heat treatment. Subsequently, the reaction solutions were collected and then condensed 15- to 30-fold using a rotary evaporator. The concentrate was adsorbed to a silica gel column (volume: 2000 mL) equilibrated with 99.5% ethanol. The column was washed with 99.5% ethanol in twice the volume of the column, so as to remove contaminating components such as unreacted glucose. Elution was then carried out sequentially with 95% ethanol in 3 times, 90% ethanol in 3 times, 85% ethanol in 3 times, and then 80% ethanol in 3 times the volume of the column. Each eluted fraction was analyzed by thin-layer chromatography, reverse phase high performance liquid chromatography, or the like. 95% to 90% ethanol eluted fractions containing target fructosyl Val-His were collected. The collected products were condensed and desiccated using a rotary evaporator, thereby obtaining approximately 3 g of a partially purified product. As a result of mass spectrometry analysis, the molecular weight of the purified product was found to be MW 416, which agreed with the molecular weight of fructosyl Val-His. Furthermore, the structure of the product was confirmed by nuclear magnetic resonance spectrum analysis. The partially purified product was adsorbed and desorbed by a standard method using an ion exchange resin to enhance the purification degree. The resultant was used for the subsequent experiments. Furthermore, a partially purified product of fructosyl Val was obtained by a method similar to that described above using Val.

Examples

(Example 1) Liberation of glycated dipeptide from glycated hexapeptide

To screen for proteases capable of efficiently excising $\alpha$-glycated dipeptide, proteases listed in Table 1 were caused to act on $\alpha$-glycated hexapeptide (fructosyl Val-His-Leu-Thr-Pro-Glu; produced by PEPTIDE INSTITUTE, INC.). The amounts of the thus generated products were determined using fructosyl peptide oxidases or a fructosyl amino acid oxidase.

<Preparation of protease reaction sample>

1.8 mM $\alpha$-glycated hexapeptide: 12 $\mu$l
20 mg/ml Protease solution (the solution was prepared at as high a concentration as possible when this concentration was unable to be achieved, or the same concentration was used when protease was in a liquid state): 8 $\mu$l
100 mM Potassium phosphate buffer pH 8.0 (pH was appropriately changed according to the optimum protease pH): 4 $\mu$l

The above ingredients were mixed well and then allowed to react at 37°C for 2 hours. The resultant was subjected to heat treatment at 90°C for 3 minutes and then centrifuged, thereby obtaining a supernatant that was separated into protease reaction samples. Furthermore, similar procedures were carried out using distilled water instead of a substrate, thereby preparing a blank sample.

<Solution for determining the reaction of glycated dipeptide and glycated amino acid in protease reaction sample>

100 mM Potassium phosphate buffer pH 8.0
45 mM 4AA
0.5 mM TOOS
1 U/ml POD (produced by KIKKOMAN CORPORATION)
0.1 U/ml Fructosyl peptide oxidase or fructosyl amino acid oxidase

145 $\mu$l of the above reaction solution for determining the amount of glycated dipeptide and glycated amino acid were apportioned into wells of a microtiter plate. 5 $\mu$l of the above protease reaction sample was added to and sufficiently mixed well with the solution. The resultants were subjected to determination at 555 nm ($A_0$). Subsequently, incubation was carried out at 30°C for 20 minutes and the resultants were subjected to determination at 555 nm ($A_1$). Similar procedures were carried out using the blank sample instead of protease reaction samples, thereby obtaining $A_0$ blank and $A_1$ blank. The following formula represents the action of protease on the $\alpha$-glycated hexapeptide as a change in absorbance.

$$\Delta A = (A_1 - A_0) - (A_1 \text{ blank} - A_0 \text{ blank})$$

In addition, the following four oxidases were used in the above reaction solution for determining the amount of a glycated product: FPOX-C and FPOX-E (both produced by KIKKOMAN CORPORATION) as fructosyl peptide oxidases; FAOX (produced by KIKKOMAN CORPORATION) as fructosyl amino acid oxidase; and FLOD (produced by Asahi Kasei Corporation). These oxidases differ in substrate specificity. Specifically, while FPOX-C and FPOX-E act on both fructosyl Val-His and fructosyl Val, FAOX and FLOD act only on fructosyl Val. Hence, it was predicted that when fructosyl Val-His was excised by the above protease treatment, changes in absorbance would be observed for FPOX-C and FPOX-E. It was also predicted that if fructosyl Val were to be excised, changes in absorbance would be observed for FPOX-C, FPOX-E, FAOX, and FLOD.

Table 1 shows the results (units; mAbs).

| Protease name | | Origin | FPOX-C | FPOX-E | FAOX | FLOD |
|---|---|---|---|---|---|---|
| IP enzyme | KIKKOMAN | Aspergillus | 38 | 51 | 1 | 2 |
| AO protease | KIKKOMAN | | 63 | 46 | 0 | 0 |
| Peptidase | KIKKOMAN | | 65 | 50 | 1 | 0 |
| Molsin | KIKKOMAN | | 5 | 8 | 1 | 1 |
| Protease A5 | KYOWAKASEI | | 21 | 14 | 0 | 0 |
| Umamizyme | Amano | | 37 | 20 | 0 | 0 |
| Protease A | Amano | | 78 | 51 | 0 | 0 |
| Protease M | Amano | | 85 | 63 | 0 | 4 |
| Protease P | Amano | | 126 | 89 | 2 | 1 |

(continued)

| Protease name | | Origin | FPOX-C | FPOX-E | FAOX | FLOD |
|---|---|---|---|---|---|---|
| Sumizyme MP | Shin Nihon Chemical | | 142 | 105 | 0 | 0 |
| Sumizyme LP-20 | Shin Nihon Chemical | | 71 | 52 | 0 | 1 |
| Sumizyme LPL | Shin Nihon Chemical | | 8 | 6 | 0 | 0 |
| Sumizyme AP | Shin Nihon Chemical | | 5 | 5 | 2 | 2 |
| Proteinase 6 | Fluka | | 119 | 87 | 0 | 0 |
| Peptidase R | Amano | Rhizopus | 65 | 50 | 0 | 1 |
| Newlase F | Amano | | 2 | 1 | 0 | 0 |
| Dispase | Roche | Bacillus | 63 | 32 | 1 | 2 |
| Subtilisin | Boehringer | | 10 | 6 | 1 | 0 |
| Proteinase N | Fluka | | 114 | 82 | 0 | 0 |
| Proteinase Type VII | Sigma | | 12 | 10 | 2 | 2 |
| Proteinase, Bacterial Subtilisin | Fluka | | 41 | 33 | 1 | 2 |
| Protease N | Amano | | 63 | 44 | 0 | 0 |
| Proleather FG-F | Amano | | 4 | 4 | 0 | 0 |
| Protease S | Amano | | 129 | 87 | 0 | 0 |
| Proteinase Type X | Sigma | | 73 | 53 | 0 | 1 |
| Thermolysin | DAIWA KASEI | | 73 | 51 | 2 | 2 |
| Pronase E | Kaken Pharmaceutical | | 31 | 11 | 1 | 3 |
| Neutral protease | TOYOBO | | 132 | 0 | | 0 |
| Pronase | Boehringer | Streptomyces | 35 | 17 | 4 | 3 |
| Proteinase Type XIV | Sigma | | 143 | 84 | 2 | 0 |
| Alkaline protease | TOYOBO | | 39 | 29 0 | 0 | 0 |
| Proteinase K | Roche | Tritirachium | 79 | 73 | 2 | 1 |
| Proteinase K | Wako | | 36 | 22 | 0 | 0 |
| AP-I | Takara | Achromobacter | 1 | 0 | 0 | 1 |
| Lysylendpeptidase | Wako | | 3 | 1 | 2 | 1 |
| Asp-N | Takara | Pseudomonas | 0 | 0 | 0 | 0 |
| Pfu protease | Takara | Pyrococcus | 3 | 2 | 0 | 0 |
| Deblocking aminopeptidase | Takara | | 0 | 1 | 0 | 0 |
| PD enzyme | KIKKOMAN | Penicillium | 1 | 2 | 1 | 1 |
| Aminopeptidase T | Wako | Thermus | 0 | 0 | 2 | 0 |
| V8 protease | Takara | Staphylococcus | 1 | 2 | 1 | 2 |
| V8 protease | Wako | | 3 | 0 | 0 | 0 |
| Glu-C | Boehringer | | 4 | 2 | 3 | 1 |
| Papain | Roche | Papaya | 90 | 69 | 3 | 1 |
| Papain | Wako | | 51 | 30 | 0 | 0 |
| Papain | Sigma | | 52 | | 0 | 1 |
| Papain W40 | Amano | | 49 | 27 21 | 1 | 0 |
| Papain | Asahi | | 55 | 27 | 2 | 0 |
| Ficin | Sigma | Fig | 15 | 7 | 3 | 1 |
| Bromelain F | Amano | Pineapple | 4 | 2 | 0 | 0 |
| Bromelain | Sigma | | 4 | 2 | 1 | 0 |
| Pancreatin | Wako | Swine pancreas | 28 | 17 | 0 | 1 |

(continued)

| Protease name | | Origin | FPOX-C | FPOX-E | FAOX | FLOD |
|---|---|---|---|---|---|---|
| Cathepsin B | Sigma | Bovine spleen | 21 | 16 | 1 | 1 |
| Cathepsin C | Sigma | Bovine spleen | 0 | 1 | 2 | 1 |
| Cathepsin D | Sigma | Bovine spleen | 2 | | 0 | 1 |
| Elastase | Boehringer | Swine pancreas | 1 | 1 | 1 | 0 |
| m-calpain | Nacalai | Swine kidney | 1 | 1 | 1 | 0 |
| $\mu$-calpain | Nacalai | Swine erythrocyte | 1 | 1 | 1 | 0 |
| Trypsin | Wako | Swine pancreas | 2 | 2 | 1 | 2 |
| Trypsin | Sigma | Bovine pancreas | 2 | 1 | 0 | 0 |
| Trypsin | Takara | Bovine pancreas | 5 | 1 | 0 | 1 |
| $\alpha$-chymotrypsin | Sigma | Bovine pancreas | 1 | 0 | 1 | 2 |
| $\alpha$-chymotrypsin | Sigma | Bovine pancreas | | 2 | 2 | 1 |
| Pepsin | Wako | Swine | 1 | 2 | 2 | 1 |
| Pepsin | Sigma | Swine | 0 | 0 | 0 | 0 |
| Aminopeptidase M | Roche | Swine pancreas | 1 | 1 | 1 | 1 |
| Leucine aminopeptidase | Sigma | Swine | 3 | 3 | 1 | 1 |
| Carboxypeptidase A | Sigma | Bovine pancreas | 0 | 0 | 0 | 0 |
| Carboxypeptidase B | Sigma | Swine pancreas | 3 | 3 | 1 | 2 |
| N acylaminoacyl-peptide hydrolase | Takara | Swine liver | 0 | 0 | 0 | 1 |

When the activity of proteases is evaluated through detection of the generated product using FAOX or FLOD (detection of fructosyl Val), changes in absorbance obtained for all the protease cases were approximately 0. This suggests that various proteases that have been said to excise fructosyl Val from glycated protein or glycated peptide have extremely weak activity of excising fructosyl Val. Such various proteases are leucine aminopeptidase, deblocking aminopeptidase, N-acylaminoacyl-peptide hydrolase, and cathepsin C (all disclosed in JP Patent Publication (Kokai) No. 2002-315600 A); aminopeptidase, carboxypeptidase, trypsin, chymotrypsin, subtilisin, proteinase K, papain, cathepsin B, pepsin, thermolysin, lysylendpeptidase, proleather, and bromelain (all disclosed in International Patent Publication No. 97/13.872 pamphlet); and serine carboxypeptidase (disclosed in JP Patent Publication (Kokai) No. 2001-57897 A).

In contrast, when detection was carried out using FPOX-C or FPOX-E (detection of fructosyl Val-His), strong changes in absorbance were observed in the cases of IP enzyme, AO protease, peptidase, protease A5, umamizyme, protease A, protease M, protease P, sumizyme MP, sumizyme LP-20, and proteinase 6 as *Aspergallus*-derived enzymes; peptidase R as a *Rhizopus*-enzyme; dispase, subtilisin, proteinase N, proteinase Type VII, proteinase (Bacterial), protease N, proteinase Type X, thermolysin, pronase E, and neutral protease as *Bacillus*-derived enzymes; pronase, proteinase Type XIV, and alkaline protease as *Streptomyces*-derived enzymes; proteinase K as a *Tritirachium*-derived enzyme, papain and ficin as plant-derived enzymes; and pancreatin and cathepsin B as animal-derived enzymes.

Further weaker changes in absorbance were observed in the cases of molsin, sumizyme LPL, and sumizyme AP as *Aspergillus*-derived enzymes; proleather FG-F as a *Bacillus*-derived enzyme; Glu-C as a *Staphylococcus*-derived enzyme; and bromelain as a plant-derived enzyme. As described above, it was shown that $\alpha$-glycated dipeptide can be effectively excised from $\alpha$-glycated hexapeptide through the above protease treatment.

(Example 2) Activity of protease to excise glycated dipeptide with short reaction time

To screen for proteases capable of efficiently excising $\alpha$-glycated dipeptide within shorter reaction times, experiments similar to those in Example 1 were carried out without changing the various conditions thereof. The reaction time period for protease was shortened to 5 minutes from 2 hours, however. The amounts of $\alpha$-glycated dipeptide and $\alpha$-glycated amino acid were determined after reaction. The results are represented by the following equation (similar to that in Example 1)

$$\Delta A = (A_1 - A_0) - (A_1 \text{ blank} - A_0 \text{ blank})$$

The results are also summarized in Table 2 (units; mAbs).

Table 2

| Protease name | | Origin | FPOX-C | FPOX-E | FAOX | FLOD |
|---|---|---|---|---|---|---|
| AO protease | KIKKOMAN | Aspergillus | 24 | 19 | 2 | 0 |
| Peptidase | KIKKOMAN | | 0 | 1 | 0 | 0 |
| Molsin | KIKKOMAN | | 1 | 0 | 0 | 1 |
| Protease P | Amano | | 119 | 91 | 0 | 1 |
| Sumizyme MP | Shin Nihon Chemical | | 124 | 95 | 1 | 0 |
| Dispase | Roche | Bacillus | 98 | 71 | 0 | 1 |
| Proteinase N | Fluka | | 105 | 84 | 0 | 0 |
| Protease S | Amano | | 119 | 90 | 0 | 0 |
| Proteinase K | Roche | Tritirachium | 26 | 20 | 2 | 2 |
| Papain | Roche | Papaya | 89 | 64 | 0 | 0 |

As a result of comparison with *Aspergillus*-derived proteases (AO protease, peptidase, and molsin) disclosed in JP Patent Publication (Kokai) No. 2003-235585 A, *Aspergillus*-derived proteases (protease P and sumizyme MP) showed changes in absorbance that were approximately 5 times higher than that in the case of AO protease, *Bacillus*-derived proteases (dispase, proteinase N, and protease S) showed changes that were 4 to 5 times higher than that in the case of AO protease, *Tritirachium*-derived protease (proteinase K) showed changes that were almost equivalent to that in the case of AO protease, and plant-derived protease (papain) showed changes that were approximately 4 times higher than that in the case of AO protease. Hence, it was shown that the use of the above proteases enables more efficient excising of glycated dipeptide within shorter time periods. This suggests that determination of the amount of glycated protein or glycated peptide is possible with higher sensitivity within shorter time periods

(Example 3) Confirmation of liberated glycated dipeptide by HPLC (outside the scope of the invention)

The above $\alpha$-glycated hexapeptide was dissolved in water, so as to prepare 5 mM solutions. 0.01 mL of a protease solution (papain (produced by Roche), ficin (produced by Sigma-Aldrich Corporation), or dispase (produced by Roche)) and 0.09 mL of a buffer (0.1 M) were added to and mixed with 0.1 mL of each of the above solutions. Thus, protease treatment was carried out. The above mixtures were allowed to react at 37°C for 60 minutes. Subsequently, each treated solution was appropriately condensed and diluted and then subjected to HPLC determination. For HPLC (reverse phase high performance liquid chromatography), CAPCEL-PAK C-18 (produced by Shiseido Co., Ltd.) was used. The resultants were eluted with gradient using 0.1% TFA (trifluoroacetic acid)/water-0.1% TFA/30% acetonitrile as an eluant. As a standard substance, an $\alpha$-glycated dipeptide (fructosyl Val-His) was used. As a result, it was confirmed that $\alpha$-glycated dipeptide (fructosyl Val-His) had been liberated through treatment with each protease (papain, ficin, or dispase) in the treated solution.

(Example 4) Determination of the amount of glycated hexapeptide using protease and oxidase (outside the scope of the invention)

It was examined by the following experiment whether or not the amount of glycated hexapeptide can be determined using the protease screened for in Examples 1 and 2 and fructosyl peptide oxidase.

<Protease reaction>

1.8 mM $\alpha$-glycated hexapeptide
3 U/ml Papain (produced by Roche): 8 $\mu$l
Water (to a total volume of 24 $\mu$l)

The amount of the above $\alpha$-glycated hexapeptide to be used for reaction was varied in 0, 1, 2, 3, 4, 5, 6, and 7 $\mu$l samples 8 $\mu$l of papain and water were added to a total volume of 24 $\mu$l. The solution was allowed to react at 37°C for 10 minutes, subjected to heat treatment at 90°C for 5 minutes, and then subjected to centrifugation, thereby obtaining a supernatant as a protease reaction sample. Furthermore, similar procedures were carried out using distilled water instead of a substrate, thereby preparing a blank sample.

<Solution for determining the reaction of glycated dipeptide in protease reaction sample>

100 mM Potassium phosphate buffer pH 8.0
45 mM 4AA
0.5 mM TOOS
1 U/ml POD (produced by KIKKOMAN CORPORATION)
0.1 U/ml Fructosyl peptide oxidase, FPOX-C (produced by KIKKOMAN CORPORATION)

145 $\mu$l of a solution for determining the reaction of the above glycated dipeptide was apportioned into wells of a microtiter plate. 5 $\mu$l of the above protease reaction sample was added to each well. After sufficient mixing, the resultants were subjected to determination at 555 nm ($A_0$). Subsequently, incubation was carried out at 30°C for 20 minutes, followed by determination at 555 nm ($A_1$). Furthermore, similar procedures were carried out using the blank sample instead of protease reaction samples, thereby obtaining $A_0$ blank and $A_1$ blank. The following formula was obtained when the action of the protease on $\alpha$-glycated hexapeptide was represented by a change in absorbance.

$$\Delta A = (A_1 - A_0) - (A_1 \, \text{blank} - A_0 \, \text{blank})$$

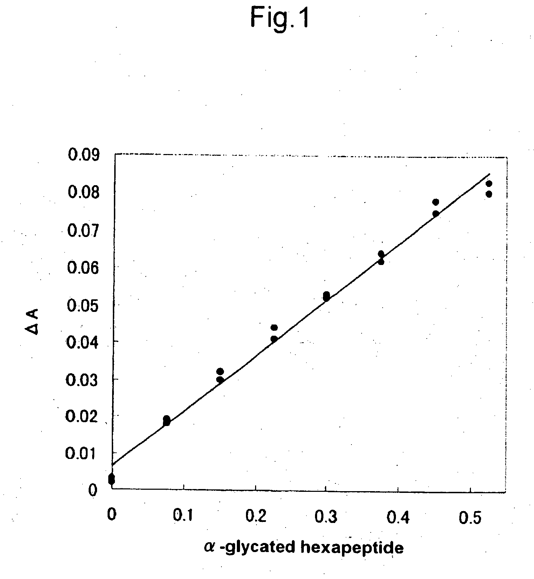

Fig. 1 shows the results of determining the amount of $\alpha$-glycated hexapeptide at each concentration. As shown in Fig. 1, there was a linear correlation between $\Delta A$ and the concentrations of $\alpha$-glycated hexapeptide. Specifically, it was shown that excision of $\alpha$-glycated dipeptide through the above protease treatment enables to determine the amount of $\alpha$-glycated hexapeptide in a highly precise manner within a short time period.

As described above, it was suggested that regarding $\alpha$-glycated hexapeptide, which is known to be obtained through endoprotease Glu-C treatment for HbA1c, enzymatically more convenient HbA1c determination is made possible by carrying out the protease treatment of the present invention for $\alpha$-glycated hexapeptide without carrying out capillary electrophoresis or mass spectroscopy.

(Example 5) Production of $\alpha$-glycated dipeptide through treatment of HbA1c with Glu-C and neutral protease (outside the scope of the invention)

It was confirmed by the following experiment whether or not $\alpha$-glycated dipeptide was generated by causing Glu-C and neutral protease to act on HbA1c.

<Protease reaction>

14.4% HbA1c solution (produced by KYOWA MEDEX CO., LTD.): 44 $\mu$l
0.5 mg/ml Glu-C (produced by Wako Pure Chemical Industries, Ltd.): 36 $\mu$l
150 mM Ammonium acetate (pH 4.0): 8 $\mu$l

The mixed solution was incubated at 37°C overnight. Subsequently, 352 $\mu$l of neutral protease (2.4 U/ml dispase; produced by Roche) was added to the solution, and then the solution was stirred Furthermore, the solution was incubated at 37°C overnight The solution was then subjected to heat treatment at 92°C for 5 minutes and then centrifuged at 12,000 rpm for 5 minutes, thereby obtaining a supernatant as a sample. Furthermore, similar procedures were carried out using distilled water instead of Glu-C or dispase, thereby preparing a blank sample.

<Determination of the reaction of α-glycated dipeptide contained in protease reaction sample>

A solution for determining the reaction was prepared as follows. In addition, FAOX and catalase in R1 were used for removing contaminating glycated amino acids in a sample.

| | |
|---|---|
| R1: | |
| 50 mM | POPSO buffer (pH 7.5) (produced by DOJINDO LABORATORIES) |
| 5 U/ml | FAOX (produced by KIKKOMAN CORPORATION) |
| 300 U/ml | Catalase (produced by KIKKOMAN CORPORATION) |
| R2: | |
| 100 mM | Tris-HCl buffer (pH7.5) (produced by Nacalai Tesque, Inc.) |
| 0.1 mM | DA-64 (produced by Wako Pure Chemical Industries, Ltd.) |
| 10 mM | Ca-EDTA (produced by DOJINDO LABORATORIES) |
| 150 U/ml | POD (produced by KIKKOMAN CORPORATION) |
| 0.15% | $NaN_3$ (produced by Wako Pure Chemical Industries, Ltd.) |
| 40 U/ml | Fructosyl peptide oxidase, FPOX-E (produced by KIKKOMAN CORPORATION) |

216 μl of R1 was added to 30 μl of the sample. After 5 minutes of treatment, 80 μl of R2 was added to and mixed with the solution. The solution was allowed to react at 37°C for 5 minutes. The increased absorbance (ΔAbs) (difference between absorbance determined before and the same determined after reaction with R2) was determined at 750 nm using a Hitachi autoanalyser (model 7070). Thus, the increased absorbace was found to be 0.007. In contrast, ΔAbs was 0 in the case of the blank sample. Furthermore, a similar result was obtained even when FPOX-C (produced by KIKKOMAN CORPORATION) had been used as fructosyl peptide oxidase.

Accordingly, it was confirmed that α-glycated dipeptide is generated through treatment of HbA1c with Glu-C and neutral protease. It was also confirmed that the amount of the generated α-glycated dipeptide can be determined using FPOX-E and -C.

(Example 6) Production of α-glycated dipeptide through treatment of HbA1c with neutral protease (outside the scope of the invention)

Glu-C and neutral protease were caused to act on HbA1c in Example 5. In this example, it was examined by the following experiment whether or not α-glycated dipeptide was generated by causing neutral protease alone to act thereon.

<Protease reaction>

14.4% HbA1c solution (produced by KYOWA MEDEX CO., LTD): 88 μl
2.4 U/ml Neutral protease (dispase; produced by Roche): 352 μl

The mixed solution was incubated at 37°C overnight. The solution was then subjected to heat treatment at 92°C for 5 minutes and then centrifuged at 12,000 rpm for 5 minutes, thereby obtaining a supernatant as a sample (the HbA1c amount used herein was twice that used in Example 5). Furthermore, similar procedures were carried out using distilled water instead of the neutral protease, thereby preparing a blank sample.

<Determination of the reaction of α-glycated dipeptide contained in protease reaction sample>

R1 and R2 used herein were the same as those used in Example 5.

216 μl of R1 was added to 30 μl of the sample. After 5 minutes of treatment, 80 μl of R2 was added to and mixed with the solution. The solution was allowed to react at 37°C for 5 minutes. As a result, the increased absorbance (ΔAbs) (difference between absorbance determined before and the same determined after reaction with R2) determined at 750 nm was found to be 0.007. In contrast, ΔAbs was 0 in the case of the blank sample. The HbA1c amount used in this protease treatment was twice that used in Example 5. However, ΔAbs (=0.007) equivalent to that in Example 5 was observed. Furthermore, a similar result was obtained even when FPOX-C (produced by KIKKOMAN CORPORATION) was used as fructosyl peptide oxidase. Accordingly, it was confirmed that α-glycated dipeptide is generated through treatment of HbA1c with neutral protease alone. It was also confirmed that the generated α-glycated dipeptide can be detected using FPOX-E and -C.

(Example 7) Determination of the amount of HbA1c using FPOX (outside the scope of the invention)

HbA1c control (calibrator for determining the amount of Determiner HbAlc; produced by KYOWA MEDEX CO., LTD.) was dissolved in a diluted solution of a specimen (produced by KYOWA MEDEX CO., LTD.). Five HbA1c solutions varying in concentrations (0.0%, 4.1%, 7.8%, 11.3%, and 14.4%) were prepared. The following procedures were carried out using these solutions.

<Protease reaction>

Each HbA1c solution: 44 μl
2.4 U/ml Neutral protease (dispase; produced by Roche): 176 μl

The mixed solutions were incubated at 37°C overnight. The solutions were subjected to heat treatment at 92°C for 5 minutes and then centrifuged at 12,000 rpm for 5 minutes, thereby obtaining supernatants as samples. Furthermore, similar procedures were carried out using distilled water instead of the neutral protease, thereby preparing a blank sample.

<Determination of the reaction of α-glycated dipeptide contained in protease reaction sample>

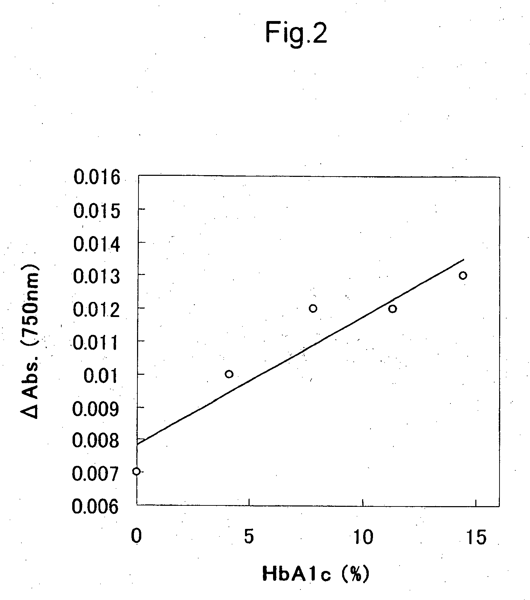

R1 and R2 used herein were the same as those used in Example 5.
216 μl of R1 was added to 30 μl of each sample. After 5 minutes of treatment, 80 μl of R2 was added to and mixed with the solution. The solution was allowed to react at 37°C for 5 minutes. As a result, the increased absorbance (ΔAbs) (difference between absorbance determined before and the same determined after reaction with R2) was determined at 750 nm. Fig. 2 shows the relationship between HbA1c concentrations and ΔAbs as obtained by this method. Fig. 2 shows that there is a correlation between HbA1c concentrations and the generated amounts of hydrogen peroxide. In addition, ΔAbs obtained by similar procedures was always 0 in the blank samples wherein distilled water had been added instead of the neutral protease to the HbA1c solutions with various concentrations.

Industrial Applicability

According to the present invention, a method for producing α-glycated dipeptide is provided, which enables the simple, rapid, and efficient production of α-glycated dipeptide from glycated protein or glycated peptide. Also disclosed is a method for determining the amount of α-glycated dipeptide, which enables to determine the amount of α-glycated dipeptide in a highly precise manner within a short time period. Such determination method is particularly effective in determination of the amount of N-terminal-glycated peptide, protein, protein subunits, and the like such as HbA1c.

Brief Description of the Drawings

Fig. 1    shows the results of determining the amount of α-glycated hexapeptide.
Fig. 2    shows the results of determining the amount of HbA1c.

**Claims**

1.  A method for producing α-glycated dipeptide, which comprises causing a protease to act on an N-terminal-glycated peptide or an N-terminal-glycated protein,
    wherein said protease is at least one selected from the group consisting of *Aspergillus* protease P, *Aspergillus* protease M, *Aspergillus* protease A, *Bacillus* dispase, *Bacillus* proteinase N, and *Bacillus* protease S.

2.  The method for producing α-glycated dipeptide according to claim 1, wherein the N-terminal-glycated peptide is fructosyl Val-His-Leu-Thr-Pro-Glu.

3. The method for producing α-glycated dipeptide according to claim 1, wherein the N-terminal-glycated protein is glycated hemoglobin.

4. The method for producing α-glycated dipeptide according to claims 1 to 3, wherein the α-glycated dipeptide is fructosyl valyl histidine.


**Patentansprüche**

1. Verfahren zur Herstellung von α-glykiertem Dipeptid, umfassend das Einwirkenlassen einer Protease auf ein N-terminal glykiertes Peptid oder ein N-terminal glykiertes Protein,
   wobei die Protease zumindest eine aus *Aspergillus*-Protease P, *Aspergillus*-Protease M, *Aspergillus*-Protease A, Bacillus-Dispase, *Bacillus*-Proteinase N und *Bacillus*-Protease S bestehenden Gruppe ausgewählte Protease ist.

2. Verfahren zur Herstellung von α-glykiertem Dipeptid nach Anspruch 1, wobei das N-terminal glykierte Peptid Fructosyl-Val-His-Leu-Thr-Pro-Glu ist.

3. Verfahren zur Herstellung von α-glykiertem Dipeptid nach Anspruch 1, wobei das N-terminal glykierte Protein glykiertes Hämoglobin ist.

4. Verfahren zur Herstellung von α-glykiertem Dipeptid nach Anspruch 1 bis 3, wobei das α-glykierte Dipeptid Fructosylvalylhistidin ist.


**Revendications**

1. Procédé de production de dipeptides α-glyqués, qui comprend l'action d'une protéase sur un peptide glyqué au niveau de la terminaison N ou une protéine glyquée au niveau de la terminaison N,
   dans lequel ladite protéase est au moins une protéase choisie dans le groupe constitué de la protéase P de *Aspergillus,* de la protéase M de *Aspergillus,* de la protéase A de *Aspergillus,* de la dispase de *Bacillus,* de la protéinase N de *Bacillus* et de la protéase S de *Bacillus.*

2. Procédé de production de dipeptides α-glyqués selon la revendication 1, dans lequel le peptide glyqué au niveau de la terminaison N est le fructosyl Val-His-Leu-Thr-Pro-Glu.

3. Procédé de production de dipeptides α-glyqués selon la revendication 1, dans lequel la protéine glyquée au niveau de la terminaison N est l'hémoglobine glyquée.

4. Procédé de production de dipeptides α-glyqués selon les revendications 1 à 3, dans lequel le dipeptide α-glyqué est la fructosyl valyl histidine.

# Fig.1

# Fig.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5033997 B
- JP 6065300 B
- JP 3155780 A
- JP 7289253 A
- JP 8154672 A
- JP 8336386 A
- JP 5192193 A
- JP 10033177 A
- JP 10033180 A
- JP 9013872 A

- JP 2001057897 A
- JP 2000300294 A
- JP 0050579 A
- JP 0061732 A
- JP 2027012 A
- JP 2002315600 A
- JP 2001095598 A
- JP 2003235585 A
- JP 2003326224 A
- JP 2003421755 A

**Non-patent literature cited in the description**

- **KOBOLD U. et al.** *Clin. Chem.,* 1997, vol. 43, 1944-1951